# EUROPÄISCHE PATENTSCHRIFT

(11) **EP 4 247 531 B1**
(45) Veröffentlichungstag und Bekanntmachung des Hinweises auf die Patenterteilung: **11.12.2024**
(21) Anmeldenummer: 21799260.1
(22) Anmeldetag: 25.10.2021
(51) Int. Cl.: B01J 8/00, B01J 8/04, C07C 57/055, C07C 45/35, C07C 47/22, C07C 51/25

(54) **ZWEISTUFIGES HERSTELLUNGSVERFAHREN VON A,SS-ETHYLENISCH UNGESÄTTIGTEN CARBONSÄUREN UND ANLAGE HIERZU**
TWO-STAGE PREPARATION PROCESS FOR ALPHA,BETA-ETHYLENICALLY UNSATURATED CARBOXYLIC ACIDS AND PLANT FOR THE PURPOSE
PROCESSUS DE PRÉPARATION EN DEUX PHASES D'ACIDES CARBOXYLIQUES ALPHA,BETA-ÉTHYLÉNIQUEMENT INSATURÉS ET INSTALLATION À CET EFFET

(30) Priorität: 19.11.2020 EP 20208603
(43) Veröffentlichungstag der Anmeldung: 27.09.2023
(73) Patentinhaber: BASF SE, 67056 Ludwigshafen am Rhein (DE)
(72) Erfinder: GEISSLER, Tobias, 67056 Ludwigshafen (DE)
(86) Internationale Anmeldenummer: PCT/EP2021/079553
(87) Internationale Veröffentlichungsnummer: WO 2022/106158

(56) Entgegenhaltungen:
- WO-A1-02/081422
- US-A- 6 069 271

## Beschreibung

Die vorliegende Erfindung betrifft ein Verfahren zur Herstellung von α,β-ethylenisch ungesättigten Carbonsäuren durch zweistufige katalytische Gasphasenoxidation von Alkenen, insbesondere ein Verfahren zur zweistufigen Oxidation von Propylen zu Acrylsäure, und eine Anlage hierzu.

Ein derartiges zweistufiges Verfahren zur Herstellung von Acrylsäure ist per se bekannt und zum Beispiel in der WO 02/081422 A1 sowie den darin zitierten Druckschriften beschrieben. Ferner beschreibt die US 6 069 271 ein Verfahren zur Herstellung von Acrylsäure.

Zunächst wird einem ersten Reaktor Propylen und molekularer Sauerstoff zugeführt. Der Sauerstoff kann z.B. in Form von Luft eingeleitet werden. Ferner kann ein inertes Gas wie z.B. Stickstoff zugeführt werden. Im ersten Reaktor wird Propylen durch katalytische Oxidation zu Acrolein umgesetzt. Das Auslassgas des ersten Reaktors enthält somit u. a. Acrolein und nichtumgesetzten Sauerstoff. Dieses Auslassgas wird in den zweiten Reaktor eingeleitet, wobei Acrolein und Sauerstoff zu Acrylsäure umgesetzt werden.

Als Katalysatoren im ersten und zweiten Reaktor werden für die Gasphasenoxidation von Propylen zu Acrolein oder von Acrolein zu Acrylsäure geeignete Katalysator verwendet. Bisher wurde eine große Anzahl von Katalysatoren vorgeschlagen, die für die genannten Reaktionen geeignet sind. Üblicherweise werden in der Industrie Multimetalloxide von Molybdän eingesetzt.

Um das in den zweiten Reaktor eingeleitete Acrolein möglichst vollständig zu Acrylsäure umzusetzen, muss auch im zweiten Reaktor ausreichend Sauerstoff zur Verfügung stehen. Hierfür kann schon in den ersten Reaktor so viel Sauerstoff eingeleitet werden, dass nach der Umsetzung von Propylen zu Acrolein noch ausreichend Sauerstoff für die Reaktion im zweiten Reaktor verbleibt. Alternativ kann zwischen dem ersten und zweiten Reaktor Sauerstoff ergänzt werden. Da sich bei der Erhöhung des Sauerstoffanteils in einem Gemisch aus Propylen und Sauerstoff die Explosionsgefahr erhöht, ist es vorteilhaft, in den ersten Reaktor nur so viel Sauerstoff einzuleiten, wie für die Umsetzung von Propylen zu Acrolein erforderlich ist. Anschließend wird zwischen dem ersten und zweiten Reaktor so viel Sauerstoff ergänzt, wie für die Umsetzung des Acroleins zu Acrylsäure erforderlich ist.

Es wurde festgestellt, dass im Betrieb einer Anlage, die dieses Verfahren ausführt, im zweiten Reaktor ein Druckverlust beim Durchleiten des Reaktionsgases. Durch das Betreiben der Synthese bei erhöhtem Druck treten Einbußen in der Acrylsäure-Selektivität auf. Wenn der Druckverlust ein bestimmtes Maß erreicht hat, muss der Reaktor abgeschaltet und gewartet werden. Dieser Vorgang wird auch Skimming genannt. Dieser die Wartung ist sowohl Zeit- als auch Ressourcen-fordernd, da sich die Standzeit durch Abkühlen und Aufheizen der Anlage verlängert.

Die US 6,069,271 beschreibt ein Verfahren zur Herstellung von Acrylsäure aus Propylen durch zweistufige katalytische Oxidation unter Verwendung eines einzelnen Festbett-Rohrbündelwärmeaustausch-Reaktors. Eine erste Katalysatorschicht ist im unteren Teil jedes der Reaktionsrohre vorgesehen, eine zweite Katalysatorschicht im oberen Teil davon. Dazwischen ist eine Inertschicht mit einem Hohlraumverhältnis von 40 bis 99,5% vorgesehen. Das Verfahren soll das Problem der Verstopfung der Reaktionsrohre verhindern, verursacht durch sublimierte Katalysatorkomponenten des ersten Katalysators, die vom Reaktionsgasstrom stromabwärts mitgerissen werden.

Der Erfindung liegt die Aufgabe zu Grunde, das eingangs genannte Verfahren und die eingangs genannten Anlage so weiterzubilden, dass der Wartungsaufwand verringert ist.

Erfindungsgemäß wird die Aufgabe durch ein Verfahren nach Anspruch 1 und eine Anlage nach Anspruch 6 gelöst. Vorteilhafte Ausgestaltungen und Weiterbildungen ergeben sich aus den abhängigen Ansprüchen.

Das erfindungsgemäße Verfahren zur Herstellung von α,β-ethylenisch ungesättigten Carbonsäuren durch zweistufige katalytische Gasphasenoxidation von Alkenen ist ein Verfahren, bei dem man
a) einen mindestens ein Alken enthaltenden Gasstrom in einem ersten Reaktor in Gegenwart von Sauerstoff einer ersten katalytischen Oxidationsreaktion an einem ersten Katalysator in Form eines Multimetalloxids von Molybdän unterzieht, wobei man einen mindestens einen α,β-ethylenisch ungesättigten Aldehyd enthaltenden Gasstrom erhält,
b) den mindestens einen α,β-ethylenisch ungesättigten Aldehyd enthaltenden Gasstrom durch eine Verbindungsleitung in einen zweiten Reaktor leitet und
c) den mindestens einen α,β-ethylenisch ungesättigten Aldehyd enthaltenden Gasstrom im zweiten Reaktor in Gegenwart von Sauerstoff einer zweiten katalytischen Oxidationsreaktion an einem zweiten Katalysator unterzieht, wobei man einen mindestens eine α,β-ethylenisch ungesättigte Carbonsäure enthaltenden Gasstrom erhält.

Das Verfahren ist dadurch gekennzeichnet ist, dass man den mindestens einen α,β-ethylenisch ungesättigten Aldehyd enthaltenden Gasstrom durch eine in der Verbindungsleitung angeordnete, austauschbare Struktur mit großer spezifischer Oberfläche leitet.

Der mindestens ein Alken enthaltende Gasstrom enthält ein oder mehere Alkene. Geeignete Alkene umfassen, z.B., 3 bis 5 Kohlenstoffatome pro Molekül, und sind, zum Beispiel, ausgewählt aus Propylen, 2-Methylpropylen, 1-Buten, 2-Buten oder 1-Penten. Vorzugsweise ist das Alken ausgewählt aus Propylen und 2-Methylpropylen. Propylen ist besonders bevorzugt.

Der mindestens ein Alken enthaltende Gasstrom wird einer ersten katalytischen Oxidationsreaktion in Gegenwart von Sauerstoff unterzogen. Es kann eine externe Sauerstoffquelle zugeführt werden. Geeignete externe Sauerstoffquellen umfassen, z.B., Sauerstoff, synthetische Luft und Luft. Luft ist besonders bevorzugt.

Die erste katalytische Oxidationsreaktion wird im ersten Reaktor durchgeführt. Der erste Reaktor ist ein beliebiger Reaktor für Gasphasenoxidationen. In einer bevorzugten Ausführungsform ist der erste Reaktor ein Festbett-Rohrbündelwärmeaustausch-Reaktor. Die Durchführung der Reaktion in einem Festbett-Rohrbündelwärmeaustausch-Reaktor ermöglicht eine gleichmäßige Wärmeabfuhr und einen guten Wärmeaustausch.

Üblicherweise bestehen solche Festbett-Rohrbündelwärmeaustausch-Reaktoren aus einem, in der Regel zylinderförmigen, Behälter, in welchem eine Vielzahl von Kontaktrohren (ein Rohrbündel) in üblicherweise vertikaler Anordnung untergebracht ist. Jedes dieser Kontaktrohre enthält eine Festbettanordnung des katalytisch aktiven Multimetalloxids (z.B. erster Katalysator im ersten Reaktor). Die Kontaktrohre sind mit ihren Enden in Rohrböden abdichtend befestigt und münden in je eine am oberen bzw. unteren Ende mit dem Behälter verbundene Haube. Über diese Hauben wird der die Kontaktrohre durchströmende Gasstrom zu- bzw. abgeführt, so dass jedes Kontaktrohr einer langgestreckten Reaktionseinheitszone entspricht. Üblicherweise weisen die Kontaktrohre eine Wanddicke von 1 bis 3 mm, einen Innendurchmesser von 20 bis 30 mm und eine Rohrlänge von 2 bis 4 m auf. Anwendungstechnisch zweckmäßig beläuft sich die im Behälter untergebrachte Anzahl an Kontaktrohren auf wenigstens 5.000, bevorzugt auf wenigstens 10.000. Häufig beträgt die Anzahl der im Reaktionsbehälter untergebrachten Kontaktrohre 15.000 bis 30.000. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren 35 bis 45 mm beträgt.

Ferner wird durch den die Kontaktrohre umgebenden Raum ein Wärmeaustauschmedium geleitet, um die Prozesswärme abzuleiten. Nach dem Austritt aus dem Behälter werden die Wärmeaustauschmedien, z.B. in äußeren Wärmetauschern, wieder auf ihre ursprüngliche Temperatur gebracht, bevor sie wieder in den Reaktionsbehälter eintreten. Als Wärmeaustauschmedien eignen sich insbesondere fluide Temperiermedien. Besonders günstig ist die Verwendung von Schmelzen von Salzen ("Salzbad") wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

Die erste katalytische Oxidationsreaktion wird in Gegenwart des ersten Katalysators durchgeführt. Der erste Katalysator liegt in Form eines Multimetalloxids von Molybdän vor. Derartige Katalysatoren sind an sich bekannt. In einer Ausführungsform weist das Multimetalloxid eine Stöchiometrie der allgemeinen Formel (I)

Mo₁₂BiₐFe_{b}X¹_{c}X²_{d}X³ₑX⁴_{f}Oₙ (I)

auf, worin
X¹ = Nickel und/oder Cobalt,
X² = Thallium, Samarium, ein Alkalimetall und/oder ein Erdalkalimetall,
X³ = Zink, Phosphor, Arsen, Bor, Antimon, Zinn, Cer, Blei, Vanadium, Chrom, Niob und/oder Wolfram,
X⁴ = Silicium, Aluminium, Titan und/oder Zirkonium,
a = 0,2 bis 5,
b = 0,01 bis 5,
c = 0 bis 10,
d = 0 bis 2,
e = 0 bis 8,
f = 0 bis 10, und
n eine Zahl ist, die durch die Wertigkeit und Häufigkeit der Elemente in (I) mit Ausnahme von Sauerstoff bestimmt wird.

In bevorzugten Ausführungsformen sind die stöchiometrischen Koeffizienten wie folgt:
a = 0,4 bis 2,
b = 2 bis 4,
c = 3 bis 10,
d = 0,02 bis 2,
e = 0 bis 5, und
f = 0,5 oder 1 bis 10.

X¹ ist vorzugsweise Cobalt, X² ist vorzugsweise K, Cs und/oder Sr, stärker bevorzugt K, X³ ist vorzugsweise Wolfram, Zink und/oder Phosphor, und X⁴ ist vorzugsweise Si. Besonders bevorzugt haben die Variablen X¹ bis X⁴ gleichzeitig die oben genannten Definitionen. Noch stärker bevorzugter ist es, dass alle stöchiometrischen Koeffizienten a bis f und alle Variablen X¹ bis X⁴ gleichzeitig die oben genannten vorteilhaften Definitionen aufweisen.

In einer Ausführungsform weist das katalytisch aktive Multimetalloxid eine Stöchiometrie der allgemeinen Formel (II)

[Y¹_{a'}Y²_{b'}O_{x'}]p[Y³_{c'}Y⁴_{d'}Y⁵_{e'}Y⁶_{f'}Y⁷_{g'}Y⁸_{h'}O_{y}]q (II)

auf, worin
Y¹ = nur Bismut oder Bismut und mindestens eines der Elemente Tellur, Antimon, Zinn und Kupfer,
Y² = Molybdän und/oder Wolfram,
Y³ = ein Alkalimetall, Thallium und/oder Samarium,
Y⁴ = ein Erdalkalimetall, Nickel, Kobalt, Kupfer, Mangan, Zink, Zinn, Cadmium und/oder Quecksilber,
Y⁵ = Eisen oder Eisen und mindestens eines der Elemente Vanadium, Chrom und Cer,
Y⁶ = Phosphor, Arsen, Bor und/oder Antimon,
Y⁷ = ein Seltenerdmetall, Titan, Zirkonium, Niob, Tantal, Rhenium, Ruthenium, Rhodium, Silber, Gold, Aluminium, Gallium, Indium, Silizium, Germanium, Blei, Thorium und/oder Uran,
Y⁸ = Molybdän und/oder Wolfram,
a' = 0,01 bis 8,
b' = 0,1 bis 30,
c' = 0 bis 4,
d' = 0 bis 20,
e' >0 bis 20,
f' = 0 bis 6,
g' = 0 bis 15,
h' = 8 bis 16,
x', y' Zahlen sind, die durch die Wertigkeit und Häufigkeit der Elemente in (II) mit Ausnahme von Sauerstoff bestimmt werden, und
p, q Zahlen sind, deren p/q-Verhältnis zwischen 0,1 und 10 liegt.

Besonders vorteilhafte katalytisch aktive Multimetalloxide der Stöchiometrie (II) sind diejenigen, in denen Y¹ nur Bismut ist.

Katalytisch aktive Multimetalloxide der Stöchiometrie (II) umfassen dreidimensionale Bereiche der chemischen Zusammensetzung Y¹_{a'}Y²_{b'}O_{x'}, die in einer Matrixphase der chemischen Zusammensetzung Y³_{c'}Y⁴_{d'}Y⁵_{e'}Y⁶_{f'}Y⁷_{g}Y⁸_{h'}O_{y'} dispergiert sind.

Die Herstellung derartiger Katalysatoren ist in den Dokumenten DE4407020 A1, EP575897 A1, DE3338380 A1 und EP2114562 A1 ausführlich beschrieben.

Innerhalb der Stöchiometrien der allgemeinen Formel (II) werden diejenigen bevorzugt, die der allgemeinen Formel (IIa)

[Bi_{a"}Z²b"O_{x"}]p"[Z⁸₁₂Z³_{c"}Z⁴_{d"}Fe_{e"}Z⁵_{f"}Z⁶_{g"}Z⁷_{h"}O_{y"}]q" (IIa)

entsprechen, worin
Z² = Molybdän und/oder Wolfram,
Z³ = Nickel und/oder Cobalt,
Z⁴ = Thallium, ein Alkalimetall und/oder ein Erdalkalimetall, vorzugsweise K, Cs und/oder Sr,
Z⁵ = Phosphor, Arsen, Bor, Antimon, Zinn, Cer, Vanadium, Chrom und/oder Bi,
Z⁶ = Silizium, Aluminium, Titan und/oder Zirkonium, vorzugsweise Si,
Z⁷ = Kupfer, Silber und/oder Gold,
Z⁸ = Molybdän und/oder Wolfram,
a" = 0,1 bis 1,
b" = 0,2 bis 2,
c" = 3 bis 10,
d" = 0,02 bis 2,
e" = 0,01 bis 5, vorzugsweise 0,1 bis 3,
f = 0 bis 5,
g" = 0 bis 10, vorzugsweise >0 bis 10, stärker bevorzugt 0,2 bis 10 und am meisten bevorzugt 0,4 bis 3,
h" = 0 bis 1,
x", y" Zahlen sind, die durch die Wertigkeit und Häufigkeit der Elemente in (IIa) mit Ausnahme von Sauerstoff bestimmt werden, und
p", q" Zahlen sind, deren p"/q"-Verhältnis zwischen 0,1 und 5, vorzugsweise zwischen 0,5 und 2 liegt.

Innerhalb der katalytisch aktiven Multimetalloxide der Stöchiometrie (IIa) werden solche bevorzugt, bei denen Z²_{b"} = (Wolfram)_{b"} und Z⁸₁₂ = (Molybdän)₁₂.

Die erste katalytische Oxidationsreaktion im ersten Reaktor wird bei einer Temperatur im Bereich von 200 bis 420 °C, einem Betriebsdruck im Bereich von 2 bis 3 bar absolut, und einer Verweilzeit im Bereich von 1,5 bis 2,5 Sekunden durchgeführt.

Der Produktstrom der ersten katalytischen Oxidationsreaktion umfasst, gegebenenfalls neben einem nicht umgesetzten Teil des Alkens, mindestens einen α,β-ethylenisch ungesättigten Aldehyd, der durch Partialoxidation des Alkens gebildet wurde. So führt die Partialoxidation von Propylen zu Acrolein und die Partialoxidation von 2-Methylpropylen zu Methacrolein. Vorzugsweise ist der α,β-ethylenisch ungesättigte Aldehyd ausgewählt aus Acrolein.

Der mindestens einen α,β-ethylenisch ungesättigten Aldehyd enthaltende Gasstrom wird am Ausgang des ersten Reaktors über die Verbindungsleitung abgeführt und in den zweiten Reaktor eingeleitet.

Der mindestens einen α,β-ethylenisch ungesättigten Aldehyd enthaltende Gasstrom wird einer zweiten katalytischen Oxidationsreaktion in Gegenwart von Sauerstoff unterzogen. Es kann eine externe Sauerstoffquelle zugeführt werden. Geeignete externe Sauerstoffquellen umfassen, z.B., Sauerstoff, synthetische Luft und Luft. Luft ist besonders bevorzugt.

Die zweite katalytische Oxidationsreaktion wird im zweiten Reaktor durchgeführt. Der zweite Reaktor ist ein beliebiger Reaktor für Gasphasenoxidationen. In einer bevorzugten Ausführungsform ist der zweite Reaktor ein Festbett-Rohrbündelwärmeaustausch-Reaktor. Die Durchführung der Reaktion in einem Festbett-Rohrbündelwärmeaustausch-Reaktor ermöglicht eine gleichmäßige Wärmeabfuhr und einen guten Wärmeaustausch.

Die zweite katalytische Oxidationsreaktion wird in Gegenwart eines zweiten Katalysators durchgeführt. Der zweite Katalysator liegt in Form eines Multimetalloxids von Molybdän vor. Derartige Katalysatoren sind an sich bekannt. In einer Ausführungsform weist das Multimetalloxid eine Stöchiometrie der allgemeinen Formel (III)

Mo₁₂VₐX¹_{b}X²_{c}X³_{d}X⁴ₑX⁵_{f}X⁶_{g}Oₙ (III),

auf, worin
X¹ = W, Nb, Ta, Cr und/oder Ce,
X² = Cu, Ni, Co, Fe, Mn und/oder Zn,
X³ = Sb und/oder Bi,
X⁴ = ein oder mehrere Alkalimetalle (Li, Na, K, Rb, Cs) und/oder H,
X⁵ = ein oder mehrere Erdalkalimetalle (Mg, Ca, Sr, Ba),
X⁶ = Si, Al, Ti und/oder Zr,
a = 1 bis 6,
b = 0,2 bis 4,
c = 0 bis 18, vorzugsweise 0,5 bis 18,
d = 0 bis 40,
e = 0 bis 2,
f = 0 bis 4,
g = 0 bis 40, und
n eine Zahl ist, die durch die Wertigkeit und Häufigkeit der Elemente in (III) mit Ausnahme von Sauerstoff bestimmt wird.

Vorzugsweise sind die Variablen innerhalb der angegebenen Bereiche mit der Maßgabe zu wählen, dass der molare Anteil des Elements Mo, bezogen auf die Gesamtmenge aller Elemente mit Ausnahme von Sauerstoff im Multimetalloxidmaterial (III), 20 bis 80 mol-% beträgt, das Molverhältnis von Mo, enthalten im katalytisch aktiven Multimetalloxidmaterial (III), zu V, Mo/V, enthalten im katalytisch aktiven Multimetalloxidmaterial (III), 15:1 bis 1:1, und das entsprechende Molverhältnis Mo/(Gesamtmenge von W und Nb) 80:1 bis 1:4 (und das entsprechende Molverhältnis Mo/Cu 30:1 bis 1:3, wenn das Multimetalloxidmaterial Cu enthält).

Bevorzugte Multimetalloxid-Katalysatoren (III) sind solche, bei denen
X¹ = W, Nb und/oder Cr,
X² = Cu, Ni, Co und/oder Fe,
X³ = Sb,
X⁴ = Na und/oder K,
X⁵ = Ca, Sr und/oder Ba,
X⁶ = Si, Al und/oder Ti,
a = 2,5 bis 5,
b = 0,5 bis 2,
c = 0,5 bis 3,
d = 0 bis 2,
e = 0 bis 0,2,
f = 0 bis 1,
g = 0 bis 15, und
n eine Zahl ist, die durch die Wertigkeit und Häufigkeit der Elemente in (III) mit Ausnahme von Sauerstoff bestimmt wird.

Bevorzugte Multimetalloxid-Katalysatoren entsprechen der folgenden allgemeinen Stöchiometrie (Illa)

Mo₁₂VₐX¹_{b}X²_{c}X⁵_{f}X⁶_{g}Oₙ (IIIa),

worin
X¹ = W und/oder Nb,
X² = Cu und/oder Ni,
X⁵ = Co und/oder Sr,
X⁶ = Si und/oder Al,
a = 3 bis 4,5,
b = 1 bis 1,5,
c = 0,75 bis 2,5,
f = 0 bis 0,5,
g = 0 bis 8, und
n eine Zahl ist, die durch die Wertigkeit und Häufigkeit der Elemente in (Illa) mit Ausnahme von Sauerstoff bestimmt wird.

Mo/Cu liegt zwischen 30:1 und 1:3, und das entsprechende Molverhältnis Mo/(Gesamtmenge von W und Nb) liegt zwischen 80:1 und 1:4.

Vorzugsweise sind die Variablen innerhalb der angegebenen Bereiche mit der Maßgabe zu wählen, dass der molare Anteil des Elements Mo, bezogen auf die Gesamtmenge aller Elemente mit Ausnahme von Sauerstoff in dem katalytisch aktiven Multimetalloxidmaterial (Illa), 20 bis 80 mol-% beträgt, das Molverhältnis von Mo, das in dem katalytisch aktiven Multimetalloxidmaterial (Illa) enthalten ist, zu V, Mo/V, das in dem katalytisch aktiven Multimetalloxidmaterial (Illa) enthalten ist, 15:1 bis 1:1, das entsprechende Molverhältnis Mo/Cu 30:1 bis 1:3 und das entsprechende Molverhältnis Mo/(Gesamtmenge von W und Nb) 80:1 bis 1:4.

Der erste Katalysator und zweite Katalysator kann, z.B. in Form von Pellets, Perlen oder Ringen mit einem Durchgangsloch vorliegen, die von einer Tablettiermaschine oder einer Extrusionsmaschine hergestellt werden. Andernfalls kann er in einer Form mit katalytischen Komponenten, die auf einem feuerfesten Träger abgeschieden sind, ähnlich wirksam verwendet werden.

Der erste Katalysator und zweite Katalysator sowie deren Herstellung sind zum Beispiel in US 8,232,425 B2 ausführlich beschrieben.

Die zweite katalytische Oxidationsreaktion im zweiten Reaktor wird bei einer Temperatur im Bereich von 240 bis 320 °C, einem Betriebsdruck im Bereich von 1,5 bis 2,5 bar absolut, und einer Verweilzeit im Bereich von 1 bis 1,7 Sekunden durchgeführt.

Der Produktstrom der zweiten katalytischen Oxidationsreaktion umfasst, gegebenenfalls neben einem nicht umgesetzten Teil des Alkens und/oder einem nicht umgesetzten Teil des mindestens einen α,β-ethylenisch ungesättigten Aldehyd enthaltenden Gasstroms mindestens eine α,β-ethylenisch ungesättigte Carbonsäure, die durch Partialoxidation des Aldehyds gebildet wurde. So führt die Partialoxidation von Acrolein zu Acrylsäure und die Partialoxidation von Methacrolein zu Methacrylsäure. Vorzugsweise ist die α,β-ethylenisch ungesättigte Carbonsäure ausgewählt aus Acrylsäure.

Der erzeugte mindestens eine α,β-ethylenisch ungesättigte Carbonsäure, bevorzugt Acrylsäure, enthaltende Gasstrom aus dem zweiten Reaktor enthält neben Propylen, Acrolein und Sauerstoff im Wesentlichen Acrylsäure. Die Gewinnung der Carbonsäure, bevorzugt Acrylsäure, erfolgt üblicherweise durch Absorption oder fraktionierte Destillation.

Die Absorption von Acrylsäure erfolgt in einer zur Absorption von Acrylsäure geeigneten Absorptionsflüssigkeit, wie z.B. Diphenyl, Diphenylether, Dimethylphthalat, Ethylhexansäure, N-Methylpyrrolidon, Paraffinfraktionen oder Gemische davon; oligomere Acrylsäuren, wie Di-, Tri- und Tetraacrylsäure enthaltende Gemische, oder Wasser. Die Absorptionsflüssigkeit wird mit dem mindestens eine α,β-ethylenisch ungesättigte Carbonsäure enthaltenden Gasstrom in einer Absorptionskolonne im Gegenstrom, z.B. nach Abkühlung mittels Wärmetauscher bei einer Temperatur von 100 bis 180 °C, in Kontakt gebracht. Als Absorptionskolonne eignen sich z.B. Füllkörper-, Packungs-, Ventilboden- oder Glockenbodenkolonnen. Die mit Acrylsäure beladene Absorptionsflüssigkeit enthält in der Regel flüchtige Verunreinigungen, wie Wasser, Acrolein, Formaldehyd, Ameisensäure und/oder Essigsäure. Diese können durch Strippen mit einem Strippgas, z.B. Stickstoff oder Luft, in einer Desorptionskolonne im Gegenstrom zumindest teilweise entfernt werden. Die Gewinnung der Rohacrylsäure erfolgt üblicherweise durch rektifikative Abtrennung bei vermindertem Druck, z.B. 0,04 bis 0,1 bar, z.B. in einer Füllkörper- oder Bodenkolonne. Die Rohacrylsäure wird als Kopfprodukt oder über einen Seitenabzug im oberen Bereich der Rektifikationskolonne abgenommen, wobei die Absorptionsflüssigkeit zweckmäßigerweise zurückgeführt und wieder zur Absorption verwendet wird. Bei Verwendung von Wasser als Absorptionsflüssigkeit wird die Rohacrylsäure aus der wässrigen Acrylsäurelösung durch Extraktion in einer Extraktionskolonne im Gegenstrom mit einem Extraktionsmittel, wie z.B. Ethylacetat, Butylacetat, Ethylacrylat, 2-Butanon oder Gemische davon, und anschließender Destillation des Extrakts isoliert.

Zur fraktionierten Kondensation wird der mindestens eine α,β-ethylenisch ungesättigte Carbonsäure enthaltende Gasstrom auf 100 bis 180 °C gekühlt und zweckmäßigerweise in den unteren Bereich einer Kolonne mit trennwirksamen Einbauten eingeleitet. Beim Aufsteigen innerhalb der Kolonne kann man eine Mittelsiederfraktion als Rohacrylsäurefraktion über einen geeignet angebrachten Fangboden abnehmen. Diese Rohacrylsäurefraktion kann in einer Kristallisation weiter gereinigt werden. Das Kristallisationsverfahren unterliegt keiner Beschränkung.

Bei allen Aufarbeitungsschritten können in an sich bekannter Weise Stabilisatoren und/oder Polymerisationsinhibitoren für Acrylsäure zugesetzt werden. Ein geeigneter Stabilisator ist z.B. Phenothiazin. Geeignete Polymerisationsinhibitoren sind z.B. Hydrochinon, Hydrochinonmonomethylether, p-Nitrosophenol, tert-Butylphenole oder Gemische davon.

Alle Anlagen-Bauteile, die im beschriebenen Verfahren mit Reaktionsgasen in Kontakt kommen, sind aus unter den vorherrschenden Reaktionsbedingungen Essigsäure-, Acrolein- oder Acrylsäure-beständigen Materialien gefertigt.

Es wurde festgestellt, dass die im ersten Katalysator verwendeten Molybdän-Verbindungen die Tendenz aufweisen, unter den im ersten Reaktor herrschenden Reaktionsbedingungen zu sublimieren. Die ausgetragenen Katalysatorbestandteile gelangen bei herkömmlichen Anlagen mit zwei Reaktoren mit dem Reaktionsgas aus dem ersten Reaktor über die Verbindungsleitung in den zweiten Reaktor. Dort setzen sie sich z.B. auf inerten Füllkörpern in der Vorheizzone und/oder auf dem zweiten Katalysator ab. Hierdurch verengt sich der freie Querschnitt im zweiten Reaktor (fouling) und der Druckverlust beim Durchleiten des Reaktionsgases durch den zweiten Reaktor steigt an. Durch das Betreiben der Synthese bei erhöhtem Druck treten daher Einbußen in der Acrylsäure-Selektivität auf. Wenn der Druckverlust ein bestimmtes Maß erreicht hat, muss der Reaktor abgeschaltet und die oberste Schicht des Bettes oder der inerten Füllkörper ausgetauscht werden (Skimming).

Erfindungsgemäß wird dieser Wartungsaufwand dadurch verringert, dass der mindestens einen α,β-ethylenisch ungesättigten Aldehyd enthaltende Gasstrom, der teilweise ausgetragene Katalysator-Bestandteile des ersten Katalysators mitführt, nach Verlassen des ersten Reaktors und vor dem Eintritt in den zweiten Reaktor durch eine in der Verbindungsleitung angeordnete, austauschbare Struktur mit großer spezifischer Oberfläche geleitet wird. Vorteilhafterweise gelangen dadurch die mit dem Reaktionsgas aus dem ersten Reaktor ausgetragenen Katalysatorbestandteile des ersten Katalysators nicht ungehindert in den zweiten Reaktor, sondern werden teilweise oder vollständig an der großen spezifischen Oberfläche der austauschbaren Struktur desublimiert.

Unter "spezifischer Oberfläche" der austauschbaren Struktur versteht man die von der austauschbaren Struktur zur Verfügung gestellte Oberfläche bezogen auf das Volumen der austauschbaren Struktur. Die "von der austauschbaren Struktur zur Verfügung gestellte Oberfläche" bezeichnet eine entsprechende, makroskopisch betrachtete Oberfläche (geometrische Oberfläche), wobei eine mögliche Rauigkeit dieser Oberfläche nicht berücksichtigt wird. Die "große spezifische Oberfläche" der austauschbaren Struktur bezeichnet insbesondere eine Oberfläche, die größer ist als die Oberfläche der Verbindungsleitung, wenn die austauschbare Struktur nicht vorhanden wäre.

Gemäß einer weiteren Ausgestaltung wird die mikroskopische Oberfläche der austauschbaren Struktur dadurch erhöht, dass die Rauigkeit dieser Oberfläche erhöht ist. Der Mittenrauwert Rₐ ist z.B. größer als 0,35 µm, bevorzugt größer als 1 µm, besonders bevorzugt größer als oder gleich 2 µm. Der Mittenrauwert Rₐ ist z. B. in einem Bereich von 2 µm bis 3 µm, insbesondere in einem Bereich von 2 µm bis 2,5 µm.

In einer Ausführungsform wird die Strömungsgeschwindigkeit des mindestens einen α,β -ethylenisch ungesättigten Aldehyd enthaltenden Gasstroms im Bereich der austauschbaren Struktur verlangsamt. Die Verlangsamung der Strömungsgeschwindigkeit wird z.B. durch eine Vergrößerung der Querschnittsfläche der Verbindungsleitung erreicht. Die Verlangsamung der Strömungsgeschwindigkeit resultiert in einer längeren Verweilzeit des mindestens einen α,β-ethylenisch ungesättigten Aldehyd enthaltenden Gasstroms in der austauschbaren Struktur. Eine längere Verweilzeit begünstigt die Desublimation der ausgetragenen Katalysatorbestandteile.

In einer Ausführungsform wird der mindestens einen α,β-ethylenisch ungesättigten Aldehyd enthaltende Gasstrom nach dem Verlassen des ersten Reaktors und vor dem Passieren der austauschbaren Struktur um 80 bis 160 °C, bevorzugt 110 bis 145 °C abgekühlt. Die Abkühlung erfolgt z.B. durch einen in der Verbindungsleitung angeordneten externen Kühler. Die externe Kühlung des mindestens einen α,β-ethylenisch ungesättigten Aldehyd enthaltenden Gasstroms, der mit dem Reaktionsgas aus dem ersten Reaktor ausgetragene Katalysatorbestandteile des ersten Katalysators enthalten kann, erleichtert die Desublimation an der großen spezifischen Oberfläche der austauschbaren Struktur.

Im erfindungsgemäßen Verfahren kann die Desublimation aus thermodynamischer Sicht durch niedrige Temperaturen des mindestens einen α,β-ethylenisch ungesättigten Aldehyd enthaltende Gasstroms oder durch niedrige Wasserkonzentrationen in besagtem Gasstrom begünstigt werden. Da bei der vorliegenden Oxidation Wasser als Nebenprodukt entsteht, kann die Wasserkonzentration nur bedingt kontrolliert werden. Die Begünstigung der Desublimation wird daher vorteilhafterweise durch Abkühlung, also bei niedrigen Temperaturen, des Gasstroms nach Verlassen des ersten Reaktors realisiert. Jedoch besteht bei zu niedrigen Temperaturen die Gefahr, dass höhersiedende Nebenkomponenten auskondensiert werden, was zur Verschmutzung von Anlagenteilen (fouling) führen kann. Im Allgemeinen werden daher die mit dem Reaktionsgas aus dem ersten Reaktor ausgetragenen Katalysatorbestandteile des ersten Katalysators teilweise an der großen spezifischen Oberfläche der austauschbaren Struktur desublimiert, während ein weiterer Teil z.B. auch an der Rohrinnenwand der Verbindungsleitung zwischen den beiden Reaktoren desublimieren kann.

Die austauschbare Struktur ist austauschbar. In einer Ausführungsform wird die austauschbare Struktur einmalig oder periodisch ausgetauscht, während der erste Reaktor (A) und/oder zweite Reaktor (B) nicht unter 150 °C abgekühlt werden. Dadurch, dass der erste Reaktor und/oder zweite Reaktor nicht vollständig abgekühlt werden, können diese thermisch nahe an den Reaktionsbedingungen gehalten werden, beispielsweise bei 150 °C und 1 bar absolut. Die Temperatur ist dabei höher als die Schmelztemperatur des die Reaktionsrohre umgebenden Temperiermediums (z.B. des Salzbads). Dadurch, dass das Salzbad in geschmolzenem Zustand gehalten wird, werden die Reaktionsbedingungen nach dem erneuten Start der Synthese sehr schnell wieder erreicht. Somit können sowohl der Zeit- als auch der Wartungsaufwand im Vergleich zu herkömmlichen Wartungs- oder Reinigungsprozessen (skimming) erheblich reduziert werden (wenige Stunden statt ca. 2 Wochen), da z.B. kein Abkühlen der Salzschmelze auf Raumtemperatur und Wiederaufheizen auf mindestens 150 °C nötig ist.

Eine bevorzugte Ausführungsform umfasst die Herstellung von Acrylsäure durch zweistufige katalytische Gasphasenoxidation von Propylen, wobei Propylen in der ersten katalytischen Oxidationsreaktion zu Acrolein oxidiert wird, welches in der zweiten katalytische Oxidationsreaktion zu Acrylsäure weiteroxidiert. Dieses Verfahren ermöglicht die effiziente Herstellung des Wertprodukts Acrylsäure, welches, z.B., großtechnische Anwendung in Polymerisationen findet.

Die Erfindung betrifft außerdem eine Anlage zur Herstellung von α,β-ethylenisch ungesättigten Carbonsäuren durch zweistufige katalytische Gasphasenoxidation von Alkenen, umfassend
a) einen ersten Reaktor, der ausgebildet ist zur Durchführung einer ersten katalytischen Oxidationsreaktion eines mindestens ein Alken enthaltenden Gasstroms in Gegenwart von Sauerstoff an einem ersten Katalysator in Form eines Multimetalloxids von Molybdän, wobei man einen mindestens einen α,β-ethylenisch ungesättigten Aldehyd enthaltenden Gasstrom erhält,
b) einen zweiten Reaktor, der ausgebildet ist zur Durchführung einer zweiten katalytischen Oxidationsreaktion des mindestens einen α,β-ethylenisch ungesättigten Aldehyd enthaltenden Gasstroms in Gegenwart von Sauerstoff an einem zweiten Katalysator, wobei man einen mindestens eine α,β-ethylenisch ungesättigte Carbonsäure enthaltenden Gasstrom erhält,
c) eine zwischen dem ersten Reaktor und dem zweiten Reaktor angeordnete Verbindungsleitung, um den mindestens einen α,β-ethylenisch ungesättigten Aldehyd enthaltenden Gasstrom in den zweiten Reaktor zu leiten, und
d) eine in der Verbindungsleitung angeordnete austauschbare Struktur mit großer spezifischer Oberfläche, durch die der mindestens einen α,β-ethylenisch ungesättigten Aldehyd enthaltende Gasstrom durchleitbar ist.

Unter "spezifischer Oberfläche" der austauschbaren Struktur versteht man die von der austauschbaren Struktur zur Verfügung gestellte Oberfläche bezogen auf das Volumen der austauschbaren Struktur. Die "große spezifische Oberfläche" der austauschbaren Struktur bezeichnet insbesondere eine Oberfläche, die größer ist als die Oberfläche der Verbindungsleitung, wenn die austauschbare Struktur nicht vorhanden wäre.

Erfindungsgemäß weist die austauschbare Struktur eine spezifische Oberfläche von mindestens 400 m²/m³, bevorzugt mindestens 600 m²/m³, auf. Der Einbau von austauschbaren Strukturen mit einer spezifischen Oberfläche von mindestens 400 m²/m³ begünstigt die Desublimation der ausgetragenen Katalysatorbestandteile und verringert dadurch den Wartungsaufwand der Anlage.

Weiterhin wird die Struktur so gewählt, dass ein möglichst geringer Druckverlust durch die Struktur auftritt. Dies kann durch die verbaute absolute Oberfläche der Struktur (S) erreicht werden. Diese ist beispielsweise mindestens 350 m².

In einer Ausführungsform weist die austauschbare Struktur eine spezifische Oberfläche pro Gasvolumen von mindestens 600 m²/m³, bevorzugt mindestens 800 m²/m³ auf. Unter "spezifischer Oberfläche pro Gasvolumen" der austauschbaren Struktur versteht man die von der austauschbaren Struktur zur Verfügung gestellte Oberfläche, an welcher der durchströmende Gasstrom, z.B. der Acrolein enthaltende Gasstrom, direkt vorbeiströmt und dabei mit der Oberfläche der Struktur in Berührung kommt, und zwar bezogen auf das Hohlraumvolumen der Struktur, welches von dem Gasstrom durchströmt wird.

Der Einbau von austauschbaren Strukturen mit einer spezifischen Oberfläche pro Gasvolumen von mindestens 600 m²/m³ begünstigt die Desublimation der ausgetragenen Katalysatorbestandteile und verringert dadurch den Wartungsaufwand der Anlage.

In einer Ausführungsform ist die austauschbare Struktur eine strukturierte Packung. Unter dem Begriff der "strukturierten Packung" versteht man Einbauten wie z.B. Metallplatten (Böden), Metallnetze, Metallgitter, etc. Die Böden können z.B. glatt, gewellt, perforiert oder geprägt sein. Die Strömungskanäle der Metallgitter können z.B. gerade oder geneigte ausgestaltet sein. Im Allgemeinen bieten strukturierte Packungen eine große (Kontakt)-Oberfläche, jedoch einen geringen Widerstand gegen Gasströmung an.

Aus dem Stand der Technik ist die Herstellung von Acrylsäure aus Propylen bekannt, wobei in geeigneten Anlagen aus dem Reaktor ausgetragene Katalysatorbestandteile z.B. an Schüttungen, die in den Reaktionsrohren angeordnet sind, abgetrennt werden. Schüttungen stellen dabei keine strukturierten Packungen dar, sondern werden als unstrukturierte Packungen angesehen. Durch Desublimation der ausgetragenen Katalysatorbestandteile an Füllkörpern solcher Schüttungen (unstrukturierte Packung) steigt der Druckverlust stärker an als bei der Desublimation an strukturierten Packungen mit gleicher absoluter Oberfläche.

In einer Ausführungsform besteht die austauschbare Struktur aus einer Keramik. Die Keramik ist vorzugsweise eine monolithische Keramik. Monolithische Keramiken sind in der Lage, hohen Temperaturen standhalten und weisen eine hohe Erosionsbeständigkeit auf, z.B. im Vergleich zu Verbundwerkstoffen mit keramischer Matrix. Außerdem verwenden monolithische Keramiken kostengünstige Rohstoffe und können in bestimmte Formen geformt werden. Die monolithische Keramik kann, z.B., aus Siliciumdioxid (SiO₂), Siliciumnitrid (Si₃N₄), Silicium-Aluminiumoxynitrid (SiAION), Siliciumcarbid (SiC), Siliciumoxynitrid (Si₂N₂O), Aluminiumnitrid (AIN), Aluminiumoxid (Al₂O₃), Hafniumdioxid (HfO₂), Zirkoniumdioxid (ZrO₂), siliciertem Siliciumcarbid (Si-SiC) oder anderen Oxiden, Carbiden oder Nitriden oder einer Kombination davon bestehen, bevorzugt aus Siliciumdioxid (SiO₂) und/oder Aluminiumoxid (Al₂O₃).

Die austauschbare Struktur besteht insbesondere aus Blöcken, vorzugsweise aus viereckigen Blöcken. Üblicherweise enthält die austauschbare Struktur 1 bis 200, bevorzugt 50 bis 170, besonders bevorzugt 100 bis 150 Blöcke. Üblicherweise weist jeder viereckige Block Kantenlängen 100 bis 200 mm (Länge) und 100 bis 200 mm (Breite) auf. Viereckige Blöcke mit großen Kantenlängen sind bevorzugt. Die Tiefe der Blöcke ist in Strömungsrichtung des Gasstroms ausgebildet und beträgt 150 bis 350 mm. Sie ist auf den Druckverlust und die Gesamtoberfläche der austauschbaren Struktur abgestimmt. Die Blöcke sind nebeneinander angeordnet. Vorzugsweise füllen die Blöcke die Querschnittsfläche der austauschbaren Struktur aus. Unter der "Querschnittsfläche" der austauschbaren Struktur versteht man eine Fläche der austauschbaren Struktur senkrecht zur Strömungsrichtung des hindurchströmenden mindestens einen α,β-ethylenisch ungesättigten Aldehyd enthaltenden Gasstroms. Im Wesentlichen liegen zwischen den Blöcken keine Lücken vor. Gegebenenfalls können etwaige Lücken gasdicht verschlossen werden, z.B. mit einem Glasgewebe. Besonders bevorzugt füllen die Blöcke die Querschnittsfläche der austauschbaren Struktur vollständig aus.

In einer Ausführungsform umfasst die austauschbare Struktur Kanäle. Die Kanäle können insbesondere in den vorstehend genannten Blöcken gebildet sein. Die Kanäle können unterschiedliche Querschnitte aufweisen, beispielsweise rund, wabenförmig, rechteckig und quadratisch. Kanäle mit quadratischem Querschnitt sind bevorzugt. Die Längsrichtung der Kanäle ist jeweils in Strömungsrichtung des mindestens einen α,β-ethylenisch ungesättigten Aldehyd enthaltenden Gasstroms ausgerichtet. Die Oberflächen der Kanäle stellen die Oberfläche der austauschbaren Struktur bereit, an der die ausgetragenen Katalysatorbestandteile desublimieren, wodurch der Wartungsaufwand der Anlage verringert wird.

Die Querschnittsfläche der Verbindungsleitung wird vollständig durch die austauschbare Struktur ausgefüllt. Der mindestens einen α,β-ethylenisch ungesättigten Aldehyd enthaltende Gasstrom wird insbesondere durch die Kanäle der austauschbaren Struktur und somit über deren Oberfläche geleitet und kann nicht an der austauschbaren Struktur vorbeiströmen. Besonders bevorzugt kann der mindestens einen α,β-ethylenisch ungesättigten Aldehyd enthaltende Gasstrom nicht den zweiten Reaktor erreichen, ohne vorher durch die Kanäle der austauschbaren Struktur zu strömen. Das heißt, dass die Querschnittsfläche der Verbindungsleitung insbesondere keine Lücken aufweist. Die Kanäle in den Blöcken der austauschbaren Struktur werden dabei nicht als "Lücken" verstanden. Als "Lücken" versteht man, z.B., Spalten zwischen dem äußeren Ende der austauschbaren Struktur und dem Gehäuse oder Spalten zwischen zwei Blöcken, wenn diese nicht so eng nebeneinander liegen, dass sich deren Außenwände berühren.

In einer Ausführungsform weist die Verbindungsleitung ein eingebautes Gehäuse auf, in dem die austauschbare Struktur angeordnet ist. Das Gehäuse ist gasdicht aufgebaut. Die austauschbare Struktur ist insbesondere lösbar im Gehäuse montiert, sodass sie ausgetauscht werden kann. Zum Beispiel kann die austauschbare Struktur herausgenommen werden und durch eine neue austauschbare Struktur ersetzt werden.

Alternativ oder zusätzlich kann das Gehäuse von den restlichen Abschnitten der Verbindungsleitung gelöst und herausgenommen werden. Hierfür weist das Gehäuse am einen und anderen Ende jeweils einen Anschlussstutzen auf, über die das Gehäuse mit der Verbindungsleitung über Schrauben lösbar verbunden ist und mit Flanschen befestigt werden kann. In diesem Fall kann das Gehäuse zusammen mit der austauschbaren Struktur durch ein neues Gehäuse zusammen mit einer neuen austauschbaren Struktur ersetzt werden. Die austauschbare Struktur kann dann nicht lösbar im Gehäuse montiert sein. Die Anordnung der austauschbaren Struktur in einem in der Verbindungsleitung eingebauten Gehäuse ermöglicht einen einfachen und schnellen Austausch der im Gehäuse angeordneten austauschbaren Struktur, indem man eine verbrauchte im Gehäuse angeordnete austauschbare Struktur gegen eine neue Struktur oder ein neues Gehäuse mit der austauschbare Struktur ersetzt.

Üblicherweise liegt die austauschbare Struktur auf einem unter der austauschbaren Struktur im Gehäuse angebrachten Stützelement auf. Übliche Stützelemente umfassen z.B. Gitter und Roste. Die Öffnungen der Stützelemente weisen größere Querschnittsflächen auf als die Querschnittsflächen der Kanäle der strukturierten Packung. Das Stützelement dient dazu, eine Verschiebung der austauschbaren Struktur im Gehäuse oder in Richtung der Verbindungsleitung, zum Beispiel durch Herunterrutschen, zu verhindern.

In einer Ausführungsform ist die Querschnittsfläche des Gehäuses der austauschbaren Struktur größer als die Querschnittsfläche der Verbindungsleitung. Unter der "Querschnittsfläche" des Gehäuses versteht man eine Fläche des Gehäuses senkrecht zur Strömungsrichtung des hindurchströmenden mindestens einen α,β-ethylenisch ungesättigten Aldehyd enthaltenden Gasstroms. Vorzugsweise ist die Querschnittsfläche des Gehäuses der austauschbaren Struktur um mindestens 25% größer als die Querschnittsfläche der Verbindungsleitung, besonders bevorzugt um mindestens 150%. Durch die Ausführungsform, in der die Querschnittsfläche des Gehäuses der austauschbaren Struktur größer ist als die Querschnittsfläche der Verbindungsleitung, kann der Druckverlust im erfindungsgemäßen Verfahren gering gehalten werden. Ausführungsformen, in denen der Druckverlust minimiert werden kann, resultieren bei der Oxidation von Propylen zu Acrylsäure in einer höheren Selektivität von Acrylsäure und sind daher besonders bevorzugt.

Außerdem wird über größere Querschnittsflächen die zur Sublimierung der Katalysator-bestandteile zur Verfügung gestellte Oberfläche vergrößert. Insbesondere werden die Oberflächen parallel zueinander bereitgestellt, so dass selbst bei einer Anlagerung von Katalysatorbestandteilen an der Oberfläche kein Druckabfall auftritt. In der Folge bleibt der Querschnitt für den Durchtritt des Gases über längere Zeit ausreichend. Dadurch werden vorteilhafterweise die Wartungsintervalle verlängert bzw. der Wartungsaufwands der Anlage verringert.

In einer Ausführungsform umfasst die Anlage einen Kühler zur Abkühlung des mindestens einen α,β-ethylenisch ungesättigten Aldehyd enthaltenden Gasstroms nach dem Verlassen des ersten Reaktors und vor dem Passieren der austauschbaren Struktur. Die Kühlung des mindestens einen α,β-ethylenisch ungesättigten Aldehyd enthaltenden Gasstroms, der mit dem Reaktionsgas aus dem ersten Reaktor ausgetragene Katalysatorbestandteile des ersten Katalysators enthalten kann, erleichtert die teilweise oder vollständige Desublimation an der großen spezifischen Oberfläche der austauschbaren Struktur.

Sofern nicht anders angegeben, gelten die vorstehend beschriebenen Ausführungsformen, Erläuterungen und Bevorzugungen gleichermaßen für die das erfindungsgemäße Verfahren und die erfindungsgemäße Anlage.

Die vorliegende Erfindung wird nun anhand der beigefügten Zeichnungen im Detail am Beispiel der Herstellung von Acrylsäure aus Propylen erläutert.
- Figur 1: zeigt schematisch ein Ausführungsbeispiel der erfindungsgemäßen Anlage zur Herstellung von Acrylsäure durch zweistufige katalytische Gasphasenoxidation von Propylen.
- Figur 2: zeigt eine schematische, perspektivische Darstellung eines Querschnitts der austauschbaren Struktur mit großer spezifischer Oberfläche.

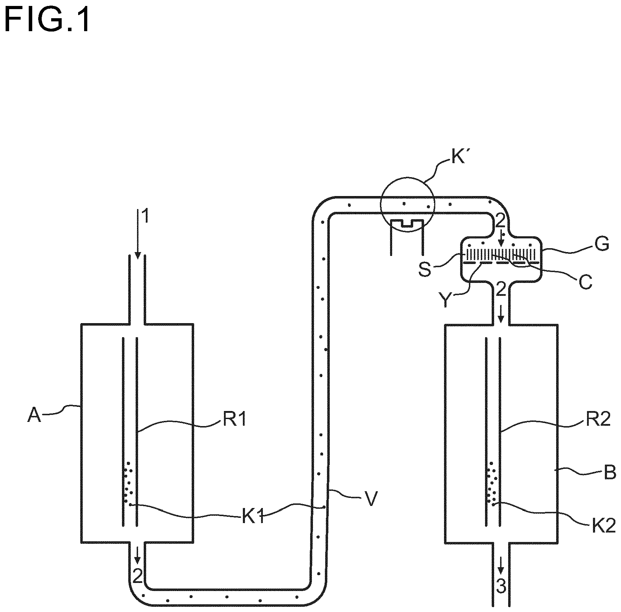

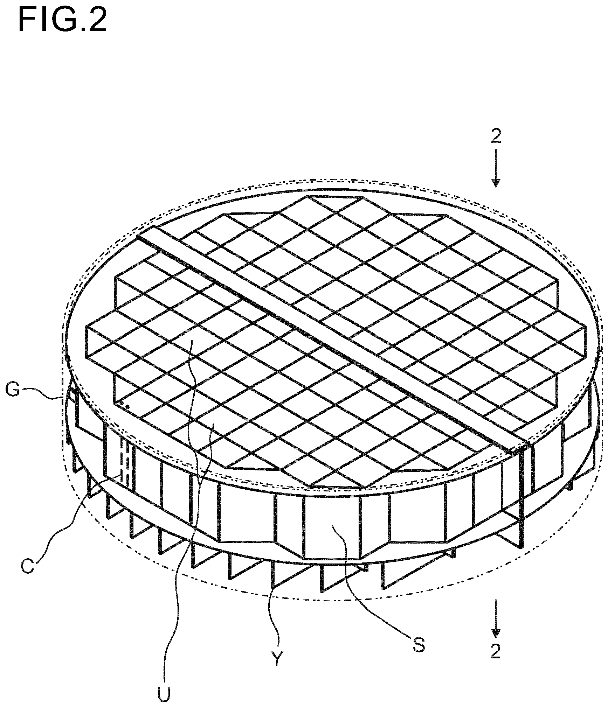

Mit Bezug zu den Figuren 1 und 2 wird ein Ausführungsbeispiel der erfindungsgemäßen Anlage erläutert:
Figur 1 zeigt eine erfindungsgemäße Anlage zur katalytischen Gasphasenoxidation von Propylen zu Acrylsäure. Die Anlage umfasst einen Salzbad-moderierten, ersten Rohrbündelwärmeaustausch-Reaktor A, der Reaktionsrohre R1 aufweist, die mit einem ersten Katalysator K1 gefüllt sind. Über eine Verbindungsleitung V ist der erste Rohrbündelwärmeaustausch-Reaktor A mit einem Salzbad-moderierten, zweiten Rohrbündelwärmeaustausch-Reaktor B verbunden. Der zweite Rohrbündelwärmeaustausch-Reaktor B enthält Reaktionsrohre R2, die mit einem zweiten Katalysator K2 gefüllt sind.

Die Verbindungsleitung V weist einen Kühler K' zwischen dem ersten Rohrbündelwärmeaustausch-Reaktor A und dem zweiten Rohrbündelwärmeaustausch-Reaktor B auf. Außerdem weist die Verbindungsleitung V ein Gehäuse G auf, in dem eine austauschbare Struktur S angeordnet ist. Das Gehäuse G ist ein Teil der Verbindungsleitung V. Es ist zwischen dem Kühler K' und dem zweiten Rohrbündelwärmeaustausch-Reaktor B in die Verbindungsleitung V eingesetzt. Die Verbindungsleitung V zwischen dem ersten Rohrbündelwärmeaustausch-Reaktor A und dem Gehäuse G hat einen Durchmesser von 1,4 m. Die Verbindungsleitung V zwischen dem Gehäuse G und dem zweiten Rohrbündelwärmeaustausch-Reaktor V hat einen Durchmesser von 1,6 m. Das die austauschbare Struktur S enthaltende Gehäuse weist einen Durchmesser von 2 m auf.

Die im Gehäuse G angeordnete austauschbare Struktur S weist Blöcke U und Kanäle C auf, die in Strömungsrichtung des hindurchströmenden Acrolein enthaltenden Gasstroms 2 ausgebildet sind. Eine schematische Darstellung eines Querschnitts der austauschbaren Struktur S ist in Figur 2 gezeigt. Die Kanäle C sind in Figur 2 als gestrichelte Linien angedeutet. Die Oberflächen der Kanäle stellen im Vergleich zur von der Verbindungsleitung alleine bereitgestellten Oberfläche eine große spezifische Oberfläche der austauschbaren Struktur zur Verfügung. Diese große spezifische Oberfläche beträgt 600 m²/m³. Die spezifische Oberfläche pro Gasvolumen beträgt 800 m²/m³. Bei der spezifischen Oberfläche handelt es sich dabei um die geometrische Oberfläche, bei der Oberflächenvergrößerungen aufgrund der Rauigkeit außer Acht gelassen werden. Der Mittenrauwert Rₐ der Struktur ist in einem Bereich von 2 µm bis 2,5 µm.

Die Blöcke der austauschbaren Struktur S liegen auf einem Rost Y im Gehäuse G auf. Sie füllen den Querschnitt des Gehäuses G und damit der Verbindungsleitung V im Wesentlichen vollständig aus. Etwaige Lücken werden beispielsweise durch Glasgewebe ausgefüllt. Das Gehäuse G weist an einem Ende und an dem anderen Ende jeweils einen Anschlussstutzen auf, über die das Gehäuse G mit anderen Abschnitten der Verbindungsleitung V über Schrauben lösbar verbunden und mit Flanschen befestigt ist.

Im Folgenden wird ein Ausführungsbeispiel des erfindungsgemäßen Verfahrens beschrieben, wobei weitere Details des Ausführungsbeispiels der erfindungsgemäßen Anlage erläutert werden:
Ein Propylen enthaltender Gasstrom 1 wird in den ersten Rohrbündelwärmeaustausch-Reaktor A eingeleitet und durchströmt die mit dem ersten Katalysator K1 gefüllten Reaktionsrohre R1. Im ersten Rohrbündelwärmeaustausch-Reaktor A wird Propylen am ersten Katalysator K1 mit Luft-Sauerstoff in einer ersten katalytischen Oxidationsreaktion zu Acrolein umgesetzt. Als erster Katalysator K1 fungiert ein Multimetalloxid von Molybdän. Ein resultierender, neben nicht umgesetztem Luft-Sauerstoff im Wesentlichen Acrolein enthaltender Gasstrom 2 verlässt, wie in Figur 1 durch den Pfeil am unteren Ende des ersten Rohrbündelwärmeaustausch-Reaktor A dargestellt, den ersten Rohrbündelwärmeaustausch-Reaktor A über Verbindungsleitung V. Bei den vorherrschenden Reaktionsbedingungen von 300 bis 400 °C und 2 bis 3 bar absolut im ersten Rohrbündelwärmeaustausch-Reaktor A sublimieren Bestandteile des ersten Katalysators K1 teilweise und werden durch den Acrolein enthaltenden Gasstrom 2 in die Verbindungsleitung V ausgetragen.

Der Acrolein enthaltende Gasstrom 2 passiert vor Erreichen des zweiten Rohrbündelwärmeaustausch-Reaktors B den Kühler K' und wird dabei von 350 °C auf 240 °C abgekühlt. Weiterhin kühlt der Acrolein enthaltende Gasstrom 2 nach Verlassen des Kühlers K` und vor Erreichen der austauschbaren Struktur S durch Wärmeverlust in der Rohrleitung von 240 °C auf 150 °C ab. Der Acrolein enthaltende Gasstrom 2 wird, wie in der Figur 1 durch die Pfeile dargestellt, durch die austauschbare Struktur S geführt, indem er die Kanäle C der Blöcke U der austauschbaren Struktur S durchströmt. Die im Acrolein enthaltenden Gasstrom 2 enthaltenen sublimitierten Bestandteile des ersten Katalysators K1 werden in der Folge teilweise oder vollständig an der Oberfläche der Kanäle C in den Blöcken U der austauschbaren Struktur S desublimiert. Der resultierende, im Wesentlichen von sublimierten Bestandteilen des ersten Katalysators K1 befreite Acrolein enthaltende Gasstrom 2 verlässt daraufhin die austauschbare Struktur S über Verbindungsleitung V (in Figur 2 nicht gezeigt).

Anschließend wird der Acrolein enthaltende Gasstrom 2 in den zweiten Rohrbündelwärmeaustausch-Reaktor B eingeleitet und durchströmt diesen, wie in Figur 1 durch den Pfeil am Reaktoreingang des zweiten Rohrbündelwärmeaustausch-Reaktor B angedeutet. Im zweiten Rohrbündelwärmeaustausch-Reaktor B wird das Acrolein in Gegenwart von Luft-Sauerstoff am zweiten Katalysator K2 einer zweiten Oxidationsreaktion unterzogen, wobei aus Acrolein Acrylsäure entsteht. Als zweiter Katalysator K2 dient ein Multimetalloxid von Molybdän. Ein resultierender Acrylsäure enthaltender Gasstrom 3 enthält neben Propylen, Acrolein und Sauerstoff im Wesentlichen Acrylsäure und wird am Ausgang des zweiten Rohrbündelwärmeaustausch-Reaktors B abgeführt.

Mit fortschreitender Abscheidung von sublimitierten Bestandteilen des ersten Katalysators K1 in den Kanälen C der austauschbaren Struktur S wird der Querschnitt der Kanäle C durch die Anlagerung der desublimitierten Bestandteile des ersten Katalysators K1 reduziert. Dies kann zu einem Druckanstieg im ersten Rohrbündelwärmeaustausch-Reaktor A führen, was einen Austausch der austauschbaren Struktur nötig macht (Wartung). Während der Lebensdauer des ersten Katalysators 1 und/oder des zweiten Katalysators 2 kann die austauschbare Struktur einmal oder periodisch ausgetauscht werden. Der Austausch erfolgt nach Unterbrechung der Synthese im ersten Rohrbündelwärmeaustausch-Reaktor A und zweiten Rohrbündelwärmeaustausch-Reaktor B durch Abstellen der Anlage (Standby). Hierfür werden die Reaktoren üblicherweise bei 1 bar absolut und etwa 150 °C gehalten. Die angegebene Temperatur ist höher als die Schmelztemperatur des Salzbads. Dadurch, dass das Salzbad in geschmolzenem Zustand gehalten wird, werden die Reaktionsbedingungen nach dem erneuten Start der Synthese sehr schnell wieder erreicht. Abkühlen der Salzschmelze auf Raumtemperatur und Re-Temperieren auf mindestens 150 °C ist im Vergleich hierzu wesentlich zeit- und ressourcen-aufwändiger.

Anschließend wird das die austauschbare Struktur S enthaltende Gehäuse G abgenommen. Hierfür werden die Schrauben der Flansche, die das Gehäuse G mit der Verbindungsleitung V verbinden, gelöst. Anschließend wird die Verbindungsleitung V an den an den Enden des Gehäuses G angebrachten Verbindungsstutzen geöffnet. Das Gehäuse G, das die verbrauchte austauschbare Struktur S enthält, wird mit einem Kran abgehoben. Anschließend wird ein neues Gehäuse G mit einer neuen austauschbaren Struktur eingesetzt und die Verbindungsleitung V wieder mit den Flanschen gasdicht verschlossen und verschraubt. In einem alternativen Ausführungsbeispiel schaltet man einen Bypass oder zwei Strukturen parallel, wobei jeweils nur eine betrieben wird und während des Betriebs auf die andere umgestellt werden kann. Anschließend kann die Synthese im ersten Rohrbündelwärmeaustausch-Reaktor A und zweiten Rohrbündelwärmeaustausch-Reaktor B wieder gestartet werden.

Die Anordnung der austauschbaren Struktur in einem in der Verbindungsleitung eingebauten Gehäuse ermöglicht einen einfachen und schnellen Austausch der im Gehäuse angeordneten austauschbaren Struktur. Zudem ist der Austausch sehr viel einfacher als in den bisher im Stand der Technik beschriebenen Verfahren. Bisher lagerten sich die ausgetragenen Katalysatorbestandteile an Oberflächen (z.B. von Schüttungen) im zweiten Rohrbündelwärmeaustausch-Reaktor B an. Beim Anstieg von Druckverlust musste folglich der Prozess unterbrochen werden, um den Reaktor abzukühlen, zu öffnen und eine Wartung in Form einer Reinigung der einzelnen Reaktionsrohre durchführen zu können. Der Wartungsaufwand der erfindungsgemäßen Anlage ist folglich um ein Vielfaches geringer.

### Bezugszeichenliste

- 1: mindestens ein Alken enthaltender Gasstrom
- 2: mindestens einen α,β-ethylenisch ungesättigten Aldehyd enthaltender Gasstrom
- 3: mindestens eine α,β-ethylenisch ungesättigte Carbonsäure enthaltender Gasstrom
- A: erster Rohrbündelwärmeaustausch-Reaktor
- B: zweiter Rohrbündelwärmeaustausch-Reaktor
- C: Kanal (von engl. channel)
- G: Gehäuse
- K': Kühler
- K1: erster Katalysator
- K2: zweiter Katalysator
- R1: Reaktionsrohr (im ersten Rohrbündelwärmeaustausch-Reaktor A)
- R2: Reaktionsrohr (im zweiten Rohrbündelwärmeaustausch-Reaktor B)
- S: austauschbare Struktur
- U: Block (von engl. unit)
- V: Verbindungsleitung
- Y: Rost

## Patentansprüche

1. Verfahren zur Herstellung von α,β-ethylenisch ungesättigten Carbonsäuren durch zweistufige katalytische Gasphasenoxidation von Alkenen, bei dem man
a) einen mindestens ein Alken enthaltenden Gasstrom (1) in einem ersten Reaktor (A) in Gegenwart von Sauerstoff einer ersten katalytischen Oxidationsreaktion an einem ersten Katalysator (K1) in Form eines Multimetalloxids von Molybdän unterzieht, wobei man einen mindestens einen α,β-ethylenisch ungesättigten Aldehyd enthaltenden Gasstrom (2) erhält,
b) den mindestens einen α,β-ethylenisch ungesättigten Aldehyd enthaltenden Gasstrom (2) durch eine Verbindungsleitung (V) in einen zweiten Reaktor (B) leitet und
c) den mindestens einen α,β-ethylenisch ungesättigten Aldehyd enthaltenden Gasstrom (2) im zweiten Reaktor (B) in Gegenwart von Sauerstoff einer zweiten katalytischen Oxidationsreaktion an einem zweiten Katalysator (K2) unterzieht, wobei man einen mindestens eine α,β-ethylenisch ungesättigte Carbonsäure enthaltenden Gasstrom (3) erhält,
**dadurch gekennzeichnet, dass** man den mindestens einen α,β-ethylenisch ungesättigten Aldehyd enthaltenden Gasstrom (2) durch eine in der Verbindungsleitung (V) angeordnete, austauschbare Struktur (S) mit großer spezifischer Oberfläche von mindestens 400 m²/m³ leitet.

2. Verfahren nach Anspruch 1, bei dem man die Strömungsgeschwindigkeit des mindestens einen α,β-ethylenisch ungesättigten Aldehyd enthaltenden Gasstroms (2) im Bereich der austauschbaren Struktur (S) verlangsamt.

3. Verfahren nach Anspruch 1 oder 2, bei dem man den mindestens einen α,β-ethylenisch ungesättigten Aldehyd enthaltenden Gasstrom (2) nach dem Verlassen des ersten Reaktors (A) und vor dem Passieren der austauschbaren Struktur (S) um 80 bis 160 °C, bevorzugt 110 bis 145 °C abkühlt.

4. Verfahren nach einem der Ansprüche 1 bis 3, bei dem man die austauschbare Struktur (S) einmalig oder periodisch austauscht, während der erste Reaktor (A) und/oder zweite Reaktor (B) nicht unter 150 °C abgekühlt werden.

5. Verfahren nach einem der Ansprüche 1 bis 4 zur Herstellung von Acrylsäure durch zweistufige katalytische Gasphasenoxidation von Propylen.

6. Anlage zur Herstellung von α,β-ethylenisch ungesättigten Carbonsäuren durch zweistufige katalytische Gasphasenoxidation von Alkenen, umfassend
a) einen ersten Reaktor (A), der ausgebildet ist zur Durchführung einer ersten katalytischen Oxidationsreaktion eines mindestens ein Alken enthaltenden Gasstroms (1) in Gegenwart von Sauerstoff an einem ersten Katalysator (K1) in Form eines Multimetalloxids von Molybdän, wobei man einen mindestens einen α,β-ethylenisch ungesättigten Aldehyd enthaltenden Gasstrom (2) erhält,
b) einen zweiten Reaktor (B), der ausgebildet ist zur Durchführung einer zweiten katalytischen Oxidationsreaktion des mindestens einen α,β-ethylenisch ungesättigten Aldehyd enthaltenden Gasstroms (2) in Gegenwart von Sauerstoff an einem zweiten Katalysator (K2), wobei man einen mindestens eine α,β-ethylenisch ungesättigte Carbonsäure enthaltenden Gasstrom (3) erhält,
c) eine zwischen dem ersten Reaktor (A) und dem zweiten Reaktor (B) angeordnete Verbindungsleitung (V), um den mindestens einen α,β-ethylenisch ungesättigten Aldehyd enthaltenden Gasstrom (2) in den zweiten Reaktor (B) zu leiten, und
d) eine in der Verbindungsleitung (V) angeordnete austauschbare Struktur (S) mit großer spezifischer Oberfläche von mindestens 400 m²/m³, durch die der mindestens einen α,β-ethylenisch ungesättigten Aldehyd enthaltende Gasstrom (2) durchleitbar ist.

7. Anlage nach Anspruch 6, bei der die austauschbare Struktur (S) eine spezifische Oberfläche von mindestens 600 m²/m³ aufweist.

8. Anlage nach Anspruch 6, bei der die austauschbare Struktur (S) eine spezifische Oberfläche pro Gasvolumen von mindestens 600 m²/m³, bevorzugt mindestens 800 m²/m³ aufweist.

9. Anlage nach einem der Ansprüche 6 bis 8, bei der die austauschbare Struktur (S) eine strukturierte Packung ist.

10. Anlage nach einem der Ansprüche 6 bis 9, bei der die austauschbare Struktur (S) aus einer Keramik, vorzugsweise aus Siliziumdioxid und/oder Aluminiumoxid, besteht.

11. Anlage nach einem der Ansprüche 6 bis 10, bei der die austauschbare Struktur (S) Kanäle (C) umfasst.

12. Anlage nach einem der Ansprüche 6 bis 11, bei der die Verbindungsleitung (V) ein eingebautes Gehäuse (G) aufweist und die austauschbare Struktur (S) in dem Gehäuse (G) angeordnet ist.

13. Anlage nach Anspruch 12, bei der die Querschnittsfläche des Gehäuses (G) der austauschbaren Struktur (S) größer ist als die Querschnittsfläche der Verbindungsleitung (V), bevorzugt um mindestens 25%, besonders bevorzugt um mindestens 150%.

14. Anlage nach einem der Ansprüche 6 bis 13, bei der der erste Reaktor (A) und/oder zweite Reaktor (B) ein Festbett-Rohrbündelwärmeaustausch-Reaktor ist.

15. Anlage nach einem der Ansprüche 6 bis 14, außerdem umfassend einen Kühler (K') zur Abkühlung des mindestens einen α,β-ethylenisch ungesättigten Aldehyd enthaltenden Gasstroms (2) nach dem Verlassen des ersten Reaktors (A) und vor dem Passieren der austauschbaren Struktur (S).

## Claims

1. A process for producing α,β-ethylenically unsaturated carboxylic acids by two-stage catalytic gas phase oxidation of alkenes, in which
a) a gas stream (1) comprising at least one alkene, in a first reactor (A) in the presence of oxygen, is subjected to a first catalytic oxidation reaction over a first catalyst (K1) in the form of a multimetal oxide of molybdenum to obtain a gas stream (2) comprising at least one α,β-ethylenically unsaturated aldehyde,
b) the gas stream (2) comprising at least one α,β-ethylenically unsaturated aldehyde is directed through a connecting conduit (V) into a second reactor (B) and
c) the gas stream (2) comprising at least one α,β-ethylenically unsaturated aldehyde, in the second reactor (B) in the presence of oxygen, is subjected to a second catalytic oxidation reaction over a second catalyst (K2) to obtain a gas stream (3) comprising at least one α,β-ethylenically unsaturated carboxylic acid,
wherein the gas stream (2) comprising at least one α,β-ethylenically unsaturated aldehyde is directed through an exchangeable structure (S) with high specific surface area of at least 400 m²/m³ which is disposed in the connecting conduit (V).

2. The process according to claim 1, in which the flow rate of the gas stream (2) comprising at least one α,β-ethylenically unsaturated aldehyde is slowed in the region of the exchangeable structure (S).

3. The process according to claim 1 or 2, in which the gas stream (2) comprising at least one α,β-ethylenically unsaturated aldehyde, after leaving the first reactor (A) and before passing through the exchangeable structure (S), is cooled by 80 to 160°C, preferably 110 to 145°C.

4. The process according to any of claims 1 to 3, in which the exchangeable structure (S) is changed once or periodically while the first reactor (A) and/or second reactor (B) is not cooled below 150°C.

5. The process according to any of claims 1 to 4 for production of acrylic acid by two-stage catalytic gas phase oxidation of propylene.

6. A plant for producing α,β-ethylenically unsaturated carboxylic acids by two-stage catalytic gas phase oxidation of alkenes, comprising
a) a first reactor (A) designed for performance of a first catalytic oxidation reaction of a gas stream (1) comprising at least one alkene in the presence of oxygen over a first catalyst (K1) in the form of a multimetal oxide of molybdenum to obtain a gas stream (2) comprising at least one α,β-ethylenically unsaturated aldehyde,
b) a second reactor (B) designed for performance of a second catalytic oxidation reaction of the gas stream (2) comprising at least one α,β-ethylenically unsaturated aldehyde in the presence of oxygen over a second catalyst (K2) to obtain a gas stream (3) comprising at least one α,β-ethylenically unsaturated carboxylic acid,
c) a connecting conduit (V) which is disposed between the first reactor (A) and the second reactor (B) in order to direct the gas stream (2) comprising at least one α,β-ethylenically unsaturated aldehyde into the second reactor (B), and
d) an exchangeable structure (S) with high specific surface area of at least 400 m²/m³ which is disposed in the connecting conduit (V), and through which the gas stream (2) comprising at least one α,β-ethylenically unsaturated aldehyde can be passed.

7. The plant according to claim 6, in which the exchangeable structure (S) has a specific surface area of at least 600 m²/m³.

8. The plant according to claim 6, in which the exchangeable structure (S) has a specific surface area per unit gas volume of at least 600 m²/m³, preferably at least 800 m²/m³.

9. The plant according to any of claims 6 to 8, in which the exchangeable structure (S) is a structured packing.

10. The plant according to any of claims 6 to 9, in which the exchangeable structure (S) consists of a ceramic, preferably of silicon dioxide and/or aluminum oxide.

11. The plant according to any of claims 6 to 10, in which the exchangeable structure (S) comprises channels (C) .

12. The plant according to any of claims 6 to 11, in which the connecting conduit (V) has an installed housing (G), and the exchangeable structure (S) is disposed within the housing (G).

13. The plant according to claim 12, in which the cross-sectional area of the housing (G) of the exchangeable structure (S) is greater than the cross-sectional area of the connecting conduit (V), preferably at least 25% greater, more preferably at least 150% greater.

14. The plant according to any of claims 6 to 13, in which the first reactor (A) and/or second reactor (B) is a fixed bed shell-and-tube heat exchange reactor.

15. The plant according to any of claims 6 to 14, also comprising a cooler (K') for cooling of the gas stream (2) comprising at least one α,β-ethylenically unsaturated aldehyde after it leaves the first reactor (A) and before it passes through the exchangeable structure (S).

## Revendications

1. Procédé de préparation d'acides carboxyliques éthyléniquement α,β-insaturés par une oxydation catalytique en phase gazeuse à deux étages d'alcènes, dans lequel
a) on soumet un flux gazeux (1) contenant au moins un alcène dans un premier réacteur (A), en présence d'oxygène, à une première réaction catalytique d'oxydation sur un premier catalyseur (K1) sous forme d'un oxyde multimétallique, un flux gazeux (2) contenant au moins un aldéhyde éthyléniquement α,β-insaturé étant obtenu,
b) on guide le flux gazeux (2) contenant au moins un aldéhyde éthyléniquement α,β-insaturé à travers une conduite de liaison (V) dans un deuxième réacteur (B) et
c) on soumet le flux gazeux (2) contenant au moins un aldéhyde éthyléniquement α,β-insaturé dans le deuxième réacteur (B), en présence d'oxygène, à une deuxième réaction catalytique d'oxydation sur un deuxième catalyseur (K2), un flux gazeux (3) contenant au moins un acide carboxylique éthyléniquement α,β-insaturé étant obtenu,
**caractérisé en ce qu'**on guide le flux gazeux (2) contenant au moins un aldéhyde éthyléniquement α,β-insaturé à travers une structure (S) remplaçable, présentant une surface spécifique élevée d'au moins 400 m²/m³, agencée dans la conduite de liaison (V).

2. Procédé selon la revendication 1, dans lequel on ralentit la vitesse d'écoulement du flux gazeux (2) contenant au moins un aldéhyde éthyléniquement α,β-insaturé dans la zone de la structure (S) remplaçable.

3. Procédé selon la revendication 1 ou 2, dans lequel on refroidit le flux gazeux (2) contenant au moins un aldéhyde éthyléniquement α,β-insaturé de 80 à 160°C, de préférence de 110 à 145°C, après qu'il a quitté le premier réacteur (A) et avant son passage dans la structure (S) remplaçable.

4. Procédé selon l'une des revendications 1 à 3, dans lequel on remplace la structure (S) remplaçable une fois ou périodiquement pendant que le premier réacteur (A) et/ou le deuxième réacteur (B) n'est/ne sont pas refroidi(s) au-dessous de 150°C.

5. Procédé selon l'une des revendications 1 à 4 pour la préparation d'acide acrylique par une oxydation catalytique en phase gazeuse à deux étages de propylène.

6. Installation pour la préparation d'acides carboxyliques éthyléniquement α,β-insaturés par une oxydation catalytique en phase gazeuse à deux étages d'alcènes, comprenant
a) un premier réacteur (A) qui est conçu pour la réalisation d'une première réaction catalytique d'oxydation d'un flux gazeux (1) contenant au moins un alcène en présence d'oxygène sur un premier catalyseur (K1) sous forme d'un oxyde multimétallique de molybdène, un flux gazeux (2) contenant au moins un aldéhyde éthyléniquement α,β-insaturé étant obtenu,
a) un deuxième réacteur (B) qui est conçu pour la réalisation d'une deuxième réaction catalytique d'oxydation du flux gazeux (2) contenant au moins un aldéhyde éthyléniquement α,β-insaturé en présence d'oxygène sur un deuxième catalyseur (K2), un flux gazeux (3) contenant au moins un acide carboxylique éthyléniquement α,β-insaturé étant obtenu,
c) une conduite de liaison (V) agencée entre le premier réacteur (A) et le deuxième réacteur (B), destinée à guider le flux gazeux (2) contenant au moins un aldéhyde éthyléniquement α,β-insaturé dans le deuxième réacteur (B) et
d) une structure (S) remplaçable, présentant une grande surface spécifique d'au moins 400 m²/m³, agencée dans la conduite de liaison (V), à travers laquelle le flux gazeux (2) contenant au moins un aldéhyde éthyléniquement α,β-insaturé peut être guidé.

7. Installation selon la revendication 6, dans laquelle la structure (S) remplaçable présente une surface spécifique d'au moins 600 m²/m³.

8. Installation selon la revendication 6, dans laquelle la structure (S) remplaçable présente une surface spécifique par volume gazeux d'au moins 600 m²/m³, de préférence d'au moins 800 m²/m³.

9. Installation selon l'une des revendications 6 à 8, dans laquelle la structure (S) remplaçable est un garnissage structuré.

10. Installation selon l'une des revendications 6 à 9, dans laquelle la structure (S) remplaçable est constituée d'une céramique, de préférence de dioxyde de silicium et/ou d'oxyde d'aluminium.

11. Installation selon l'une des revendications 6 à 10, dans laquelle la structure (S) remplaçable comprend des canaux (C).

12. Installation selon l'une des revendications 6 à 11, dans laquelle la conduite de liaison (V) présente un boîtier (G) incorporé et la structure (S) remplaçable est agencée dans le boîtier (G).

13. Installation selon la revendication 12, dans laquelle la section transversale du boîtier (G) de la structure (S) remplaçable est supérieure à la section transversale de la conduite de liaison (V), de préférence d'au moins 25%, de manière particulièrement préférée d'au moins 150%.

14. Installation selon l'une des revendications 6 à 13, dans laquelle le premier réacteur (A) et/ou le deuxième réacteur (B) est un réacteur d'échange thermique à faisceau tubulaire et à lit solide.

15. Installation selon l'une des revendications 6 à 14, comprenant en outre un refroidisseur (K') pour le refroidissement du flux gazeux (2) contenant au moins un aldéhyde éthyléniquement α,β-insaturé après qu'il a quitté le premier réacteur (A) et avant son passage dans la structure (S) remplaçable.
